# EUROPEAN PATENT APPLICATION

(11) **EP 4 772 036 A1**
(43) Date of publication of application: **08.07.2026**
(21) Application number: 24860143.7
(22) Date of filing: 04.07.2024
(51) Int. Cl.: A24F 23/02, A24B 15/16, A61K 31/465, A61K 9/00, C08J 5/18, D21H 13/08, D21H 27/00, A24B 15/18, A24B 15/24

(54) **NICOTINE POUCH AND MANUFACTURING METHOD THEREFOR**

(30) Priority: 31.08.2023 KR 20230115721
(71) Applicant: KT&G Corporation, Daedeok-gu Daejeon 34337 (KR)
(72) Inventor: KI, Sung Jong, Daejeon 34128 (KR); JEOUNG, Eun Mi, Daejeon 34128 (KR); CHA, Sung Je, Daejeon 34128 (KR); SHIN, Jun Won, Daejeon 34128 (KR)
(74) Representative: Ter Meer Steinmeister & Partner
(86) International application number: PCT/KR2024/009448
(87) International publication number: WO 2025/048219

(57) **Abstract**

The present invention relates to a nicotine pouch comprising a filler and a packaging material surrounding the filler, the filler comprising: nicotine; a release control excipient including at least one selected from the group consisting of cellulose and a sugar alcohol; a binder; a pH adjuster; and a flavoring, wherein the content of nicotine in the filler is 2.5-10 wt% with respect to the total solid content of the filler, the content of the release control excipient in the filler is 5.5-36 times that of nicotine, and the packaging material comprises at least two selected from the group consisting of pulp, a cellulose-based staple fiber, and a thermoplastic fiber.

## Description

### TECHNICAL FIELD

One or more embodiments relate to a nicotine pouch and a method of preparing the nicotine pouch.

### BACKGROUND ART

Nicotine pouches are one type of smoking article and are used in the mouth of a user to provide nicotine satisfaction to the user. For example, nicotine pouches may be used by a user placing the pouch between the upper or lower gums and lips and holding it there for a limited period of time.

A nicotine pouch includes a filler packaged in a packaging material. The packaging material holds the nicotine pouch filler in place and allows saliva to pass through the nicotine pouch filler, and a flavor and nicotine released from the nicotine pouch filler diffuse into the user's mouth.

When the amount of nicotine eluted through the packaging material is too small, it is difficult to provide the user with smoking satisfaction. On the other hand, when an elution rate of nicotine is too high, there is a problem of irritating the user or shortening the duration of the nicotine pouch due to rapid absorption of nicotine into the body.

Therefore, there is a need for a nicotine pouch in which most or all of the nicotine may be released in an appropriate amount from the nicotine pouch filler and nicotine may be effectively absorbed.

### DISCLOSURE OF THE INVENTION

### TECHNICAL GOALS

Embodiments provide a nicotine pouch having appropriate nicotine absorption and release rates and a method of preparing the nicotine pouch to meet the needs for the nicotine pouch described above.

However, goals to be achieved are not limited to those described above, and other goals not mentioned above are clearly understood by one of ordinary skill in the art from the following description.

### TECHNICAL SOLUTIONS

According to an aspect, there is provided a nicotine pouch including nicotine pouch including a filler, and a packaging material that wraps the filler, wherein the filler includes nicotine, a release control excipient including at least one or more selected from a group consisting of cellulose and sugar alcohol, a binder, a pH adjuster, and a flavoring agent, a content of the nicotine in the filler is 2.5 to 10 wt% with respect to a weight of total solid contents of the filler, a content of the release control excipient in the filler is 5.5 to 36 times the content of nicotine, and the packaging material includes at least two or more selected from a group consisting of pulp, a cellulosic staple fiber, and a thermoplastic fiber.

According to an embodiment, the cellulosic staple fiber may include one or more selected from a group consisting of rayon, viscose rayon, and lyocell.

According to an embodiment, the thermoplastic fiber may include one or more selected from a group consisting of polypropylene (PP), polyethylene (PE), polyethylene terephthalate (PET), and polypropylene/polyethylene (PP/PE).

According to an embodiment, the packaging material may further include one or more binders selected from a group consisting of polyvinyl alcohol (PVA), an acrylic binder, polylactic acid (PLA), polybutylene succinate (PBS), polyhydroxyalkanoates (PHA), a vinyl acetate copolymer, a vinyl acrylic copolymer, a styrene-butadiene copolymer, and a plant-derived natural material.

According to an embodiment, the packaging material may include 40 to 70 wt% of the pulp and 30 to 60 wt% of the thermoplastic fiber.

According to an embodiment, the packaging material may include 40 to 70 wt% of viscose rayon, 30 to 60 wt% of the thermoplastic fiber, and 0 to 15 wt% of the binder

According to an embodiment, the packaging material may be prepared by a spun-lace method, and the packaging material may include 40 to 70 wt% of viscose rayon, 30 to 60 wt% of the thermoplastic fiber, and 0 to 15 wt% of the binder.

According to an embodiment, the packaging material may be prepared by a wet laid method, and the packaging material may include 40 to 70 wt% of the pulp and 30 to 60 wt% of the thermoplastic fiber.

According to an embodiment, the packaging material may have a basis weight of 20 to 40 gsm, and a thickness of 50 to 250 µm.

According to an embodiment, the packaging material may have a porosity of 15,000 to 30,000, and a tensile strength of 1.00 to 1.50 N/mm.

According to an embodiment, a content of the cellulose in the filler may be 1 to 2 times the content of the nicotine, the content of the cellulose may be 5 to 20 wt% with respect to the weight of total solid contents of the filler, and a content of the sugar alcohol may be 50 to 70 wt% with respect to the weight of total solid contents of the filler.

According to an embodiment, the pH adjuster of the filler may include sodium bicarbonate and sodium carbonate, and a weight ratio of the sodium bicarbonate to sodium carbonate is 7:3 to 6:4.

According to an embodiment, a pH of the filler may be 8.3 to 8.5.

According to another aspect, there is provided a method of preparing the nicotine pouch described above, the method including forming a web in a form of a sheet with a fiber raw material, forming a nonwoven fabric by spraying moisture onto the web such that fibers are tangled, preparing a packaging material by drying the nonwoven fabric, and packaging a filler using the prepared packaging material.

According to still another aspect, there is provided a method of preparing the nicotine pouch described above, the method including preparing a dispersion by dispersing a fiber raw material in water, forming a web by moving the dispersion to a paper machine, preparing a packaging material by drying the formed web, and packaging a filler using the prepared packaging material.

Additional aspects of embodiments will be set forth in part in the description which follows and, in part, will be apparent from the description, or may be learned by practice of the disclosure.

### EFFECTS OF THE INVENTION

A nicotine pouch may appropriately adjust a release rate of nicotine to adjust an absorption rate of the nicotine into a user's body, thereby providing the user with satisfaction of smoking.

It should be understood that the effects of the present disclosure are not limited to the above-described effects, but are construed as including all effects that may be inferred from the configurations and features described in the following description or claims of the present disclosure.

### BRIEF DESCRIPTION OF THE DRAWINGS

These and/or other aspects, features, and advantages of the invention will become apparent and more readily appreciated from the following description of embodiments, taken in conjunction with the accompanying drawings of which:

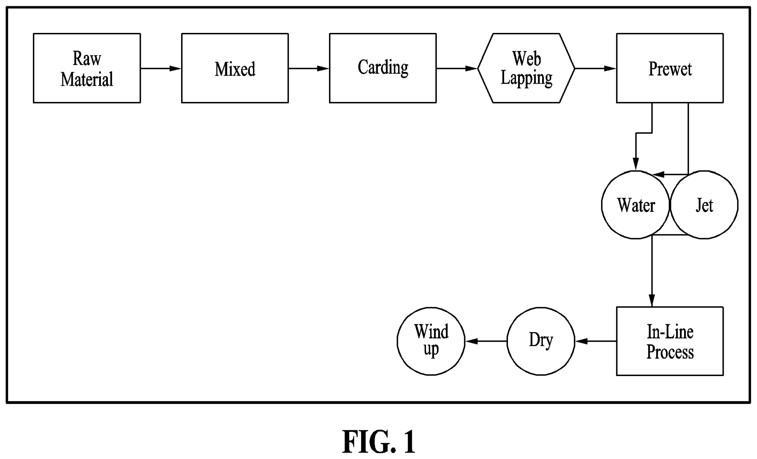

FIG. 1 is a schematic view illustrating a spun-lace process;

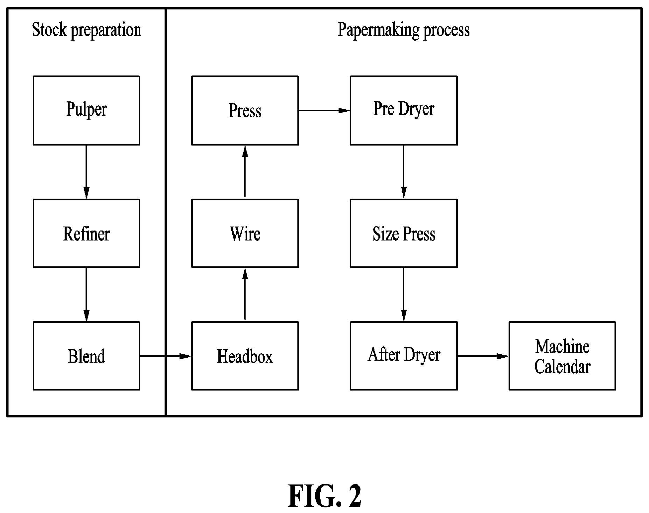

FIG. 2 is a schematic view illustrating a process of preparing a packaging material using a wet laid method;
FIG. 3 is a graph illustrating a cumulative nicotine concentration according to elution time of a nicotine pouch according to an embodiment;
FIG. 4 is a graph illustrating a nicotine concentration according to elution time of a nicotine pouch according to an embodiment;
FIG. 5 is a diagram illustrating a method of preparing a nicotine pouch filler according to an embodiment;
FIG. 6 is a diagram illustrating an elution rate according to a content of microcrystalline cellulose (MCC) of a nicotine pouch filler according to an embodiment; and
FIG. 7 is a diagram illustrating an elution rate according to added sugar alcohol of a nicotine pouch according to an embodiment.

### BEST MODE FOR CARRYING OUT THE INVENTION

Hereinafter, embodiments will be described in detail with reference to the accompanying drawings. However, various alterations and modifications may be made to the embodiments and thus, the scope of the disclosure is not limited or restricted to the embodiments. The equivalents should be understood to include all changes, equivalents, and replacements within the idea and the technical scope of the disclosure.

The terminology used herein is for the purpose of describing particular embodiments only and is not to be limiting of the embodiments. The singular forms "a," "an," and "the" are intended to include the plural forms as well, unless the context clearly indicates otherwise. It will be further understood that the terms "comprises/comprising" and/or "includes/including" when used herein, specify the presence of stated features, integers, steps, operations, elements, and/or components, but do not preclude the presence or addition of one or more other features, integers, steps, operations, elements, components and/or groups thereof.

Unless otherwise defined, all terms including technical and scientific terms used herein have the same meaning as commonly understood by one of ordinary skill in the art to which the embodiments belong. It will be further understood that terms, such as those defined in commonly-used dictionaries, should be interpreted as having a meaning that is consistent with their meaning in the context of the relevant art and will not be interpreted in an idealized or overly formal sense unless expressly so defined herein.

When describing the embodiments with reference to the accompanying drawings, like reference numerals refer to like components and a repeated description related thereto will be omitted. In the description of embodiments, detailed description of well-known related structures or functions will be omitted when it is deemed that such description will cause ambiguous interpretation of the present disclosure.

In addition, the terms first, second, A, B, (a), and (b) may be used to describe constituent elements of the embodiments. These terms are used only for the purpose of discriminating one component from another component, and the nature, the sequences, or the orders of the components are not limited by the terms.

A component, which has the same common function as a component included in any one embodiment, will be described by using the same name in other embodiments.

Unless disclosed to the contrary, the description of any one embodiment may be applied to other embodiments, and the specific description of the repeated configuration will be omitted.

It will be understood that when a certain part "includes" a certain component, the part does not exclude another component but may further include another component.

According to an embodiment, there is provided a nicotine pouch including a filler, and a packaging material that wraps the filler, wherein the filler includes nicotine, a release control excipient including at least one or more selected from a group consisting of cellulose and sugar alcohol, a binder, a pH adjuster, and a flavoring agent, a content of the nicotine in the filler is 2.5 to 10 wt% with respect to a weight of total solid contents of the filler, a content of the release control excipient in the filler is 5.5 to 36 times the content of nicotine, and the packaging material includes at least two or more selected from a group consisting of pulp, a cellulosic staple fiber, and a thermoplastic fiber.

### <Packaging material>

A packaging material may package a predetermined amount of the nicotine pouch filler. The packaging material may be packaged so that the nicotine pouch filler inside does not leak out during transportation and use after being packaged, and may be prepared so that smokers do not feel a foreign body sensation in their mouth when using it.

The packaging material may be safe for the human body, hold saliva well, have a certain strength to prevent tearing, and have sealing properties.

Among components constituting the packaging material of the nicotine pouch according to an embodiment, the pulp may include one or more selected from a group consisting of hard wood pulp and soft wood pulp, and the pulp may be desirably soft wood pulp. When the soft wood pulp is used, it may be easier to increase a tensile strength.

A content of the pulp included in the packaging material may be 0 to 70 wt%, desirably 40 to 70 wt%, and more desirably 50 to 70 wt%. When the content of the pulp exceeds the above upper limit, the content of the thermoplastic fiber is relatively small, and thus, a sufficient sealing strength may not be exhibited and stable sealing may not be formed.

Furthermore, the cellulosic staple fiber may include one or more selected from a group consisting of rayon, viscose rayon, and lyocell, and the cellulosic staple fiber may be desirably viscose rayon.

The content of cellulosic staple fiber included in the packaging material may be 0 to 70 wt%, desirably 40 to 70 wt%, and more desirably 50 to 70 wt%. If the content of the cellulosic staple fiber exceeds the upper limit, the content of thermoplastic fiber is relatively small, and thus, a sufficient sealing strength and stable sealing may not be formed.

In addition, the thermoplastic fiber may include one or more selected from a group consisting of polypropylene (PP), polyethylene (PE), polyethylene terephthalate (PET), and polypropylene/polyethylene (PP/PE).

A content of the thermoplastic fiber included in the packaging material may be 30 to 60 wt%, and desirably 35 to 50 wt%. When the content of the thermoplastic fiber is less than the lower limit, a sufficient sealing strength and stable sealing may not be formed.

The packaging material of the nicotine pouch according to an embodiment may further include one or more binders selected from a group consisting of polyvinyl alcohol (PVA), an acrylic binder, polylactic acid (PLA), polybutylene succinate (PBS), polyhydroxyalkanoates (PHA), a vinyl acetate copolymer, a vinyl acrylic copolymer, a styrene-butadiene copolymer, and a plant-derived natural material, and may be desirably PVA.

A content of the binder included in the packaging material may be 0 to 15 wt%, and desirably 5 to 15 wt%. When the content of the binder exceeds the upper limit, production of the packaging material may be impossible due to reduced manufacturing workability.

In the nicotine pouch according to an embodiment, when the packaging material is prepared by a spun-lace method, the packaging material may include 40 to 70 wt% of viscose rayon, 30 to 60 wt% of the thermoplastic fiber, and 0 to 15 wt% of the binder. When a content of viscose rayon is less than the lower limit, a surface may become rough and a porosity may be extremely reduced, and when the content of viscose rayon exceeds the upper limit, a sufficient sealing strength may not be formed. When the content of the binder exceeds the upper limit, production of the packaging material may be impossible due to reduced manufacturing workability.

Meanwhile, when the packaging material is prepared by a wet laid method, the packaging material may include 40 to 70 wt% of the pulp and 30 to 60 wt% of the thermoplastic fiber. When the content of the pulp is less than the above lower limit, the surface may become rough and the porosity may be reduced, and when the content of viscose rayon exceeds the upper limit, a sufficient sealing strength may not be formed.

The method of preparing the packaging material may include a process of forming a web and a process of bonding the web, and the process of forming the web may be a dry-laid method, a wet laid method, or a spinning method, and the dry method may be a carding method or an air ray method. The wet method may be a general paper preparing method or a partially modified preparing method, and the spinning method may be a spun-bonded or melt-blown method.

Meanwhile, the process of bonding the web may include a mechanical bonding method, a chemical bonding method, or a thermal bonding method. The mechanical bonding method may include a needle-punching method or a spun-lace method, the chemical bonding method may include a polymer dispersion method or a polymer solution method, and the thermal bonding method may include a calendering method or a hot air method, an ultrasonic method, or the like.

Desirably, the packaging material may be prepared by the spun-lace method and/or the wet laid method, and may be prepared more desirably by the wet laid method.

The spun-lace method is a process of preparing a non-woven fabric with high-pressure water using only a fiber raw material, and has the advantage of preparing a safe packaging material because there is little damage to the fiber and it does not contain additives or adhesives.

The wet laid method is similar to a general papermaking process and may be largely divided into two parts which are a stock preparation process and a papermaking process.

### 1. Stock preparation process (Pulper → Refiner → Blend)

The stock preparation process is a process just before sending a paper material to a paper machine. The pulp goes through dissociation and refining processes and a process of mixing various chemicals including the filler, goes through a selecting/dust removing process using a cleaner and a screen, and is sent to a headbox of the paper machine.

### 2. Papermaking process (Headbox → Wire → Press → Pre Dryer → Size Press → After Dryer → Machine Calendar)

The papermaking process is a process of dehydrating and drying the paper material to make paper, and includes processes such as paper layer formation, pressing and dehydration, drying, and surface treatment. A raw material ejected from the headbox is formed into paper in a wire part, and goes through a pressing and dehydration process in a press part, a residual moisture is evaporated in a dryer part, and then a thickness of the paper is adjusted in a calendar to complete a product.

When the wet laid method is used, it is easier to prepare a packaging material with a thickness, a basis weight, and a porosity suitable for nicotine pouch use, and through this, the release of nicotine may be more easily controlled.

The packaging material for the nicotine pouch prepared in the same manner as above may have a basis weight of 20 to 40 gsm and a thickness of 50 to 250 µm.

When the basis weight and the thickness of the packaging material are less than the lower limits described above, the packaging material may be damaged and small particles of the filler may flow out of the packaging material. When the basis weight and the thickness thereof exceed the upper limits described above, the amount of the filler is reduced compared to the same size, and the release of nicotine may be delayed.

In the nicotine pouch according to an embodiment, a porosity of the packaging material may be 15,000 to 30,000, and a tensile strength may be 1.00 to 1.50 N/mm.

It is necessary that the packaging material has the porosity to release the nicotine pouch filler filled inside the packaging material and has the sufficient tensile strength to prevent the pouch from bursting. Therefore, when the porosity is less than the lower limit described above, there is a possibility that active ingredients of the internal filler may not be sufficiently released due to insufficient pores. When the porosity exceeds the upper limit described above, a problem that small particles of the filler leak out to the outside may occur.

When the tensile strength is less than the lower limit described above, the pouch may burst and the internal filler may leak out to the outside.

### <Filler>

In the filler for the nicotine pouch according to an embodiment, the filler may include nicotine, a release control excipient, a binder, a pH adjuster, and a flavoring agent, the nicotine may include nicotine salt, and the nicotine salt may include at least one or more selected from a group consisting of nicotine salt benzoate, nicotine salt tartrate, nicotine salt malate, and nicotine salt salicylate.

At this time, the content of nicotine included in the filler may be 2.5 to 10 wt% with respect to a weight of total solid contents of the filler. The content of the nicotine may be determined according to a desired strength of the nicotine pouch. When the content of the nicotine is less than the range described above, it is difficult to give the smoker the satisfaction of smoking. On the other hand, when the content of the nicotine exceeds the range described above, nicotine stimulation may be excessive.

Meanwhile, the release control excipient may adjust a nicotine elution rate, and may provide a nicotine pouch that may supply an appropriate amount of nicotine when using the nicotine pouch. Desirably, a content of the release control excipient may be 5.5 to 36 times the content of the nicotine. When the content of the release control excipient is beyond the range described above, the pouch filler may dissolve quickly or a nicotine elution rate may be delayed, a core of the filler particle may not be sufficiently formed, agglomeration may be severe, or a powder may be crushed, which may make it difficult to prepare the nicotine pouch filler, and there is also a problem that the stimulation of nicotine may be too strong or too weak.

The release control excipient includes at least one selected from a group consisting of cellulose and sugar alcohol. The cellulose may function as a carrier for nicotine.

In addition, cellulose may function as a diluent with a high water holding capacity to promote the wetting of a mixture in a powder form through swelling and wicking and quickly release saliva and nicotine.

Also, the cellulose may provide usability. When the cellulose is not added, the filler for the oral pouch may come out in a dissolved form.

Specific types of the cellulose may be one or more of microcrystalline cellulose (MCC) or methyl cellulose, and a content thereof may be 1 to 2 times the content of the nicotine, and may be, specifically, 5 to 20 wt% with respect to the weight of total solid contents of the filler.

When the content of the cellulose is less than the numerical range described above, the nicotine pouch filler may dissolve quickly. On the other hand, when the content of the cellulose exceeds the numerical range described above, the nicotine elution rate may be excessively delayed due to a water-insoluble characteristic of cellulose.

The sugar alcohol is highly soluble in saliva and loosely binds to a solvent than the cellulose. Accordingly, the sugar alcohol may more rapidly release nicotine within the time of use, suppress a bitter taste, and provide a cooling effect when dissolved in the mouth.

In addition, the sugar alcohol may promote nucleation and granule growth in a high-shear wet granulation process of the nicotine pouch filler.

A content of the sugar alcohol may be 50 to 70 wt%, desirably, 60 to 70 wt% with respect to the weight of the total solid contents of the filler. When the content of the sugar alcohol is below the numerical range described above, nuclei of particles of the nicotine pouch filler may not be sufficiently formed. Meanwhile, when the content of the sugar alcohol exceeds the numerical range described above, severe agglomeration or crushing of a powder may occur, making it difficult to prepare the nicotine pouch filler.

Heat of dissolution of the sugar alcohol may be -190 J/g to -10 J/g. For example, the heat of dissolution of the sugar alcohols may be -185 J/g to -20 J/g, -125 J/g to -20 J/g, -40 J/g to -23 J/g, or -23 J/g. When the heat of dissolution of the sugar alcohol exceeds the numerical range described above, it is difficult to provide a sufficient cooling experience to the user.

A solubility of the sugar alcohol may be 10 g/100g to 80 g/100g, desirably 20 g/100g to 65 g/100g, and more desirably 60 g/100g to 65 g/100g at 20°C. When the solubility of the sugar alcohol is below the numerical range described above, it may not be sufficiently dissolved in the user's mouth. On the other hand, when the solubility of the sugar alcohol exceeds the numerical range described above, too much nicotine may be released.

The sugar alcohol may include at least one or more selected from a group consisting of erythritol, xylitol, mannitol, sorbitol, maltitol, and isomalt. However, the sugar alcohol is not limited to the examples listed above.

A type and a content of the binder included in the filler of the nicotine pouch according to an embodiment may affect the formation of a size distribution of nicotine pouch filler particles.

The binder may include at least one or more selected from a group consisting of Povidone K-25, hydroxypropylcellulose (HPC), low-substituted hydroxypropylcellulose (L-HPC), hydroxypropylmethyl cellulose (HPMC), and carboxymethyl cellulose (CMM). Desirably, the binder may be HPC. Meanwhile, the L-HPC may refer to that a content of a hydroxypropyl group is 5.0% to 16.0%.

The content of the binder may be 10 wt% or less, desirably 8 to 15 wt% with respect to the weight of the total solid contents of the filler.

When the type and content of the binder are outside the range described above, it may be difficult to secure a required particle size and may have a negative effect on initial elution.

The pH adjuster may adjust the pH of the nicotine pouch filler to a pH with which nicotine may be absorbed such that nicotine may be converted to a free-based nicotine form that may be absorbed quickly when the nicotine pouch is inserted into the mouth.

The free-base nicotine is a nonionic substance in which hydrogen ions have been removed from nicotine salt, and may be absorbed quickly through mucous membranes and have a strong impact on consumers.

In the nicotine pouch according to an embodiment, a pH of the filler may be 8.3 to 8.5. When the pH is 8.02 or more, nicotine may be converted into the free-base nicotine form, which may be quickly absorbed through the mucous membrane. Therefore, when the pH of the nicotine pouch filler satisfies the numerical range described above, the pH may be 8.02 or more when mixed with saliva (pH 6.5 to 7.7), and thus, nicotine may be easily absorbed and have a strong impact on consumers.

The pH adjuster may be at least one or more selected from a group consisting of sodium bicarbonate and sodium carbonate.

In this case, a weight ratio of the sodium bicarbonate and the sodium carbonate may be 7:3 to 6:4, desirably 6:4. When a higher proportion of sodium bicarbonate is added than the ratio described above (a ratio with a relatively lower proportion of sodium carbonate), the pH may be lowered and nicotine may not be sufficiently absorbed. On the other hand, when a lower proportion of sodium bicarbonate is added than the ratio described above (a ratio with a relatively higher proportion of sodium carbonate), the pH may become higher than the desired range and may not be suitable for elution in the mouth.

The flavoring agent in the filler may provide a taste to satisfy the smoker's preference.

The flavoring agent may include at least one or more selected from a group consisting of menthol, licorice, sucrose, fructose syrup, isosweetener, cocoa, lavender, cinnamon, cardamom, celery, fenugreek, cascarilla, sandalwood, bergamot, geranium, honey essence, rose oil, vanilla, lemon oil, orange oil, mint oil, caraway, cognac, jasmine, chamomile, cinnamon, ylang ylang, sage, spearmint, peppermint, ginger, coriander, and coffee. However, the flavoring agent is not limited to the examples described above.

The filler of the nicotine pouch according to an embodiment is not limited to including the components described above, and may optionally additionally include a humectant, an antioxidant, a preservatives, or the like.

For example, the humectant may include at least one or more of glycerin or propylene glycol.

The antioxidant may include, for example, one or more of ascorbyl palmitate and sodium ascorbate.

The preservative may include, for example, xylitol, or the like.

The filler of the nicotine pouch according to an embodiment may include 70% or more of particles with a size of 200 µm or more, and less than 30% of particles with a size of 100 µm or more and less than 200 µm. When the size of the nicotine pouch filler satisfies the numerical range described above, nicotine may be released efficiently. When there are too many small particles in the nicotine pouch filler, there may be a problem with nicotine not being eluted initially. On the other hand, when there are too many large particles in the nicotine pouch filler, there may be a problem in which all the nicotine is eluted in the beginning and nicotine is not provided to the user in a later stage of use.

### <Method of preparing nicotine pouch>

According to an embodiment, there is provided a method of preparing the nicotine pouch described above, the method including forming a web in a form of a sheet with a fiber raw material, forming a nonwoven fabric by spraying moisture onto the web such that fibers are tangled, preparing a packaging material by drying the nonwoven fabric, and packaging a filler using the prepared packaging material.

According to an embodiment, there is provided a method of preparing the nicotine pouch described above, the method including preparing a dispersion by dispersing a fiber raw material in water, forming a web by moving the dispersion to a paper machine, preparing a packaging material by drying the formed web, and packaging a filler using the prepared packaging material.

In the nicotine pouch, an inner filler may be packaged in three-sided packaging or back packaging using a packaging material, and sealing of the packaging material may be performed by ultrasonic sealing or heat sealing.

The nicotine pouch may be formed with a longitudinal seal formed along a horizontal direction of the packaging material and a cross seal formed along a vertical direction of the packaging material, and the longitudinal seal and/or cross seal may be sealed by lap sealing or fin sealing.

The packaging material may be classified into original, slim, super slim, and mini forms according to sizes and weights thereof. The packaging material may have an appropriate internal volume according to a capacity of the nicotine pouch filler to provide appropriate hardness and comfortable use.

The finally prepared nicotine pouch may be wrapped and distributed.

Hereinafter, the present disclosure will be described in more detail through examples. The following examples are described for the purpose of illustrating the present disclosure and are not intended to limit the scope of the present disclosure.

### 1. Example 1: Nicotine elution rate according to preparation method and fiber composition of packaging material

### Preparation Example 1

A packaging material for a nicotine pouch was prepared using a spun-lace method with a fiber composition of 60 wt% of viscose rayon as a cellulosic staple fiber, 30 wt% of PE as a thermoplastic fiber, and 10 wt% of PVA as a binder with respect to a total weight of the packaging material, and a nicotine pouch was prepared by packaging a filler including a filler, a pH adjuster, a binder, nicotine (including salt), and a flavoring agent with the packaging material described above.

### Preparation Example 2

A packaging material for a nicotine pouch was prepared using the spun-lace method with a fiber composition of 60 wt% of viscose rayon as a cellulosic staple fiber and 40 wt% of PE as a thermoplastic fiber with respect to a total weight of the packaging material. A nicotine pouch was prepared in the same manner as in Example 1 except for the composition of the fiber.

### Preparation example 3

A packaging material for a nicotine pouch was prepared using a wet laid method with a fiber composition of 60 wt% of soft pulp as pulp and 40 wt% of PE outer/PP inner composite yarn as a thermoplastic fiber with respect to a total weight of the packaging material, and a nicotine pouch was prepared by packaging an internal filler including a filler, a pH adjuster, a binder, nicotine (including salt), and a flavoring agent with the packaging material described above.

### Experimental Example 1

A basis weight, a thickness, a tensile strength, and a porosity of each of the nicotine pouches of Preparation Examples 1 to 3 were measured, and result values for respective measured physical properties were shown in Table 1 below.

**[Table 1]**

| | Basis weight (g/m²) | Thickness (µm) | Tensile strength MD (N/mm) | Porosity (CU) |
|---|---|---|---|---|
| Preparation Example 1 | 27.0 | 180 | 1.00 | 30000 |
| Preparation Example 2 | 40.0 | 230 | 1.50 | 25000 |
| Preparation Example 3 | 27.0 | 90 | 1.25 | 17000 |

Referring to Table 1, it may be confirmed that the basis weight, the thickness, and the porosity were measured to be low in Example 3 in which the packaging material was prepared by the wet laid method.

### Experimental Example 2

In order to compare the elution rate of each nicotine for the nicotine pouches prepared in Preparation Examples 1 to 3, an elution amount of nicotine was measured for 1 hour, at 4-minute intervals up to 20 minutes, and at 10-minute intervals from 20 minutes to 60 minutes, and a concentration of nicotine eluting, a cumulative amount of nicotine, and a cumulative nicotine concentration were calculated. The results thereof are shown in Table 2 below.

**[Table 2]**

| Product name | Time | Average amount of nicotine (mg/unit) | Cumulative nicotine amount (mg/unit) | Nicotine concentration (%) | Cumulative nicotine concentration (%) |
|---|---|---|---|---|---|
| Preparation Example 1 | 4min | 1.163 | 1.163 | 21.9% | 21.9% |
| | 8min | 1.155 | 2.318 | 21.7% | 43.6% |
| | 12min | 0.765 | 3.083. | 14.4% | 58.0% |
| | 16min | 0.515 | 3.598 | 9.7% | 67.7% |
| | 20min | 0.366 | 3.964 | 6.9% | 74.5% |
| | 30min | 0.558 | 4.522 | 10.5% | 85.0% |
| | 40min | 0.349 | 4.871 | 6.6% | 91.6% |
| | 50min | 0.253 | 5.124 | 4.8% | 96.3% |
| | 60min | 0.194 | 5.318 | 3.7% | 100.0% |
| Preparation Example 2 | 4min | 0.963 | 0.963 | 15.3% | 15.3% |
| | 8min | 1.102 | 2.065 | 17.5% | 32.8% |
| | 12min | 1.065 | 3.130 | 16.9% | 49.8% |
| | 16min | 0.783 | 3.914 | 12.5% | 62.2% |
| | 20min | 0.528 | 4.442 | 8.4% | 70.6% |
| | 30min | 0.791 | 5.233 | 12.6% | 83.2% |
| | 40min | 0.483 | 5.716 | 7.7% | 90.9% |
| | 50min | 0.320 | 6.036 | 5.1% | 96.0% |
| | 60min | 0.253 | 6.289 | 4.0% | 100.0% |
| Preparation Example 3 | 4min | 0.814 | 0.814 | 15.3% | 15.3% |
| | 8min | 0.904 | 1.718 | 17.0% | 32.3% |
| | 12min | 0.716 | 2.434 | 13.5% | 45.8% |
| | 16min | 0.596 | 3.030 | 11.2% | 57.0% |
| | 20min | 0.480 | 3.510 | 9.0% | 66.1% |
| | 30min | 0.816 | 4.326 | 15.4% | 81.4% |
| | 40min | 0.451 | 4.777 | 8.5% | 89.9% |
| | 50min | 0.307 | 5.084 | 5.8% | 95.7% |
| | 60min | 0.229 | 5.313 | 4.3% | 100.0% |

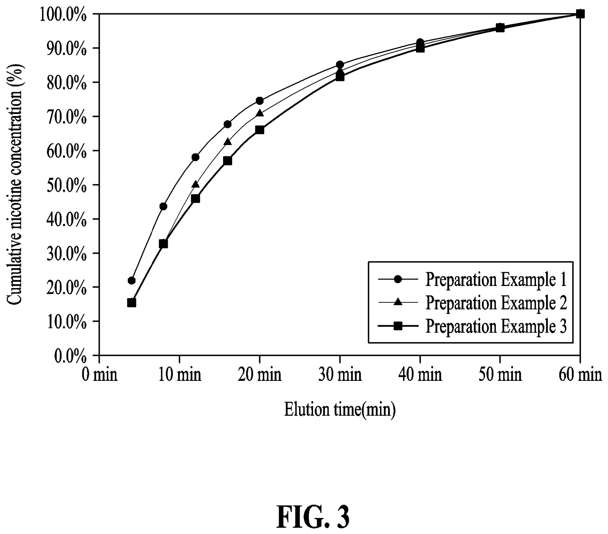

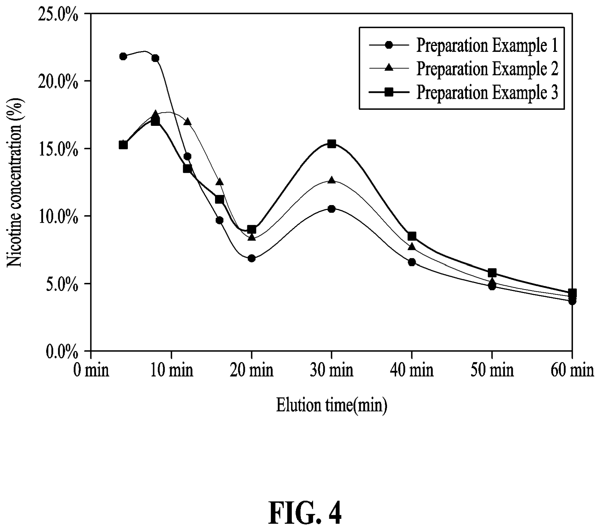

The results of the above table are shown as a graph of the cumulative nicotine concentration according to an elution time in FIG. 3, and shown as a graph of the nicotine concentration according to the elution time in FIG. 4.

Referring to Table 2 and FIGS. 3 and 4, it may be confirmed that the nicotine pouch prepared by the wet laid method has a slowest nicotine elution rate and a smallest difference between the concentrations of nicotine released over time. This is assumed to be because a moisture absorption and release rate of the pulp is lower than that of viscose rayon.

### 2. Example 2: Nicotine elution rate according to content of MCC

### Preparation Example 4

A nicotine pouch including a nicotine pouch filler was prepared. At this time, the nicotine pouch filler included 4.5 wt% of nicotine, 20 wt% of MCC, 50 to 60 wt% of sugar alcohol, 10 wt% of a pH adjuster, and 8 wt% of a binder.

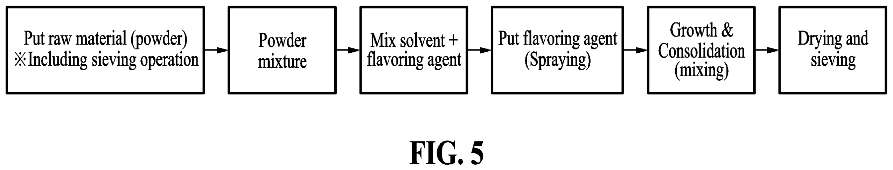

A specific preparation method is shown in FIG. 5.

### Comparative Example 1

A nicotine pouch was prepared in the same manner as in Preparation Example 4, except that the content of MCC was 25 wt%.

### Comparative Example 2

A nicotine pouch was prepared in the same manner as in Preparation Example 4, except that the content of MCC was 40 wt%.

### Comparative Example 3

A nicotine pouch was prepared in the same manner as in Preparation Example 4, except that the content of MCC was 55 wt%.

### Experimental Example 3

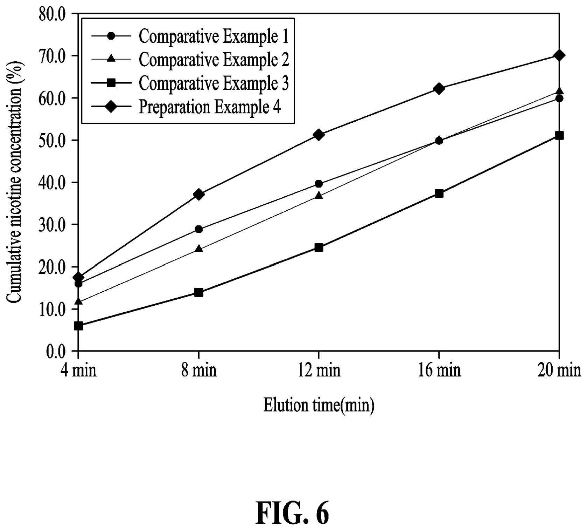

Nicotine elution rates of Preparation Example 4 and Comparative Examples 1 to 3 were analyzed. The nicotine elution rate was evaluated by measuring a content of released nicotine after leaving Preparation Examples and Comparative Examples in 1 liter of a phosphate buffer maintained at a temperature of 37°C and a pH of 7.4 for 1 minute using a USP paddle device. The results thereof are shown in FIG. 6 and Table 3.

**[Table 3]**

| | Preparation Example 4 | Comparative Example 1 | Comparative Example 2 | Comparative Example 3 |
|---|---|---|---|---|
| MCC content (wt%) | 20 | 25 | 40 | 55 |
| Elution rate after first 4 minutes (%) | 17.5 | 16.1 | 11.7 | 6.1 |
| Elution rate after 20 minutes (%) | 70.1 | 59.9 | 61.7 | 51.2 |

Preparation Example 4 showed a highest elution rate, with an elution rate of 17.5% after the first 4 minutes. However, as the content of MCC increased, the elution rate tended to decrease after the first 4 minutes due to the water-insoluble characteristic of MCC (Comparative Examples 1 to 3).

### 3. Example 3: Nicotine elution rate according to addition of sugar alcohol

### Preparation Example 5

A nicotine pouch including a nicotine pouch filler was prepared. The nicotine pouch filler included nicotine, MCC, and maltitol, a content of maltitol was 70 wt% with respect to a weight of total solid contents, and a content of MCC was 5 wt% with respect to the weight of total solid contents.

### Preparation Example 6

A nicotine pouch was prepared in the same manner as in Preparation Example 5, except that the content of maltitol was 55 wt% with respect to the weight of total solid contents, and accordingly, the content of MCC was 20 wt% with respect to the weight of total solid contents.

### Experimental Example 4

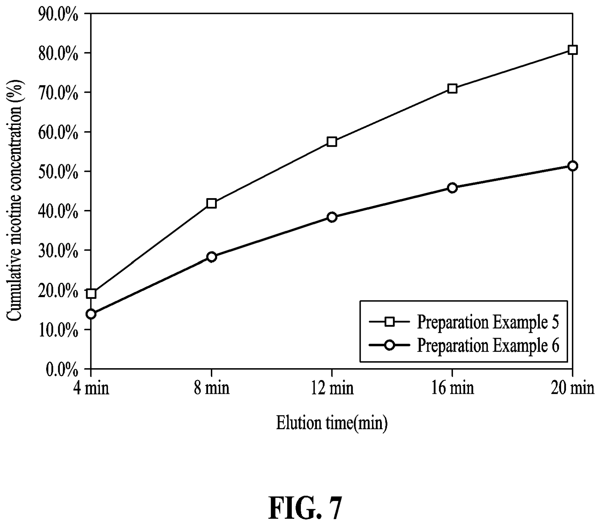

Nicotine elution rates of Preparation Examples 5 and 6 were analyzed. The nicotine elution rate was evaluated in the same manner as in the experimental examples described above. The results thereof are shown in FIG. 7.

As a result, Preparation Example 5 showed a nicotine elution rate of 20% after the first 4 minutes and 80% after 20 minutes, and Preparation Example 6 showed a nicotine elution rate of 14% after the first 4 minutes and 51.6% after 20 minutes.

The fact that Preparation Example 5 showed a higher elution rate compared to Preparation Example 6 is determined to be due to the higher content of sugar alcohol.

### 4. Example 4: Distribution of particle size and nicotine elution rate according to type of binder

### Preparation Example 7

A nicotine pouch including a nicotine pouch filler was prepared. The nicotine pouch filler included nicotine, MCC, maltitol, a binder, a pH adjuster, and a flavoring agent, the binder was HPC, and a content of the binder was 8 wt% with respect to the weight of total solid contents.

The nicotine pouch filler was prepared using a dry binder addition method. After first mixing nicotine, MCC, maltitol, a pH adjuster, and a flavoring agent, a solvent, that is water alone, water + alcohol, or alcohol alone, was sprayed, and a granulation process was performed to prepare the nicotine pouch filler.

### Preparation Example 8

A nicotine pouch was prepared in the same manner as in Preparation Example 7, except that Povidone K-25 was used as a binder and alcohol was applied as a solvent.

### Preparation Example 9

A nicotine pouch was prepared in the same manner as in Preparation Example 7, except that L-HPC was used as a binder.

### Experimental Example 5

Nicotine elution rates of Preparation Examples 5, 7 to 9 were analyzed. The nicotine elution rate was evaluated in the same manner as in the experimental examples described above. The results thereof are shown in Table 4.

**[Table 4]**

| Classification | | Preparation Example 5 | Preparation Example 7 | Preparation Example 8 | Preparation Example 9 |
|---|---|---|---|---|---|
| Elution rate after first 4 minutes (%) | | 20 | 17.5 | 15.3 | 13.8 |
| Elution rate after 20 minutes (%) | | 80 | 70.1 | 61.9 | 55.3 |
| Distribution of particle size (%) | Size of 200 µm or more | 70 | 66.7 | 50.5 | 61.8 |
| | Size of 50 µm or less | None | None | 2.2 | 1.6 |

In Preparation Examples 5 and 7, more than 66.7% of nicotine pouch filler particles with a size of 200 µm or more were distributed, and there were no nicotine pouch filler particles with a size of 50 µm or less. Preparation Examples 5 and 7 had higher nicotine elution rates not only initially but also after 20 minutes compared to Preparation Examples 8 and 9.

### 5. Example 5: Free-base nicotine generation rate according to pH range and proportion of pH adjuster

### Preparation Example 10

A nicotine pouch including a nicotine pouch filler was prepared. The nicotine pouch filler included a pH adjuster, and sodium bicarbonate and sodium carbonate were used as the pH adjuster in a ratio of 6:4.

### Preparation Example 11

A nicotine pouch was prepared in the same manner as in Preparation Example 10, except that sodium bicarbonate and sodium carbonate were used as the pH adjuster in a ratio of 7:3.

### Comparative Example 4

A nicotine pouch was prepared in the same manner as in Preparation Example 10, except that sodium bicarbonate and sodium carbonate were used as the pH adjuster in a ratio of 8:2.

### Experimental Example 6

A pH and a free-base nicotine generation rate of the nicotine pouch fillers of Preparation Examples 10 and 11 and Comparative Example 4 were investigated, and the results thereof are shown in Table 5.

**[Table 5]**

| Classification | pH | free-base nicotine generation rate (%) |
|---|---|---|
| Preparation Example 10 | 8.8 | 85% to 90% |
| Preparation Example 11 | 8.4 | 76% |
| Comparative Example 4 | 7.6 | 24% |

Unlike Comparative Example 4, Preparation Examples 10 and 11 showed results in which the pH of saliva was 8.8 and 8.4, respectively, and the free-base nicotine generation rate was 76% or more. It is confirmed that, when saliva with a pH of 6.5 to 7.7 is introduced, the pH becomes 8.02 or more, and thus, more free-base nicotine is generated.

From the above experimental examples, it is expected that using the nicotine pouch filler described in the claims may easily make nicotine to be eluted and promote absorption of the eluted nicotine.

While the embodiments are described with reference to drawings, it will be apparent to one of ordinary skill in the art that various alterations and modifications in form and details may be made in these embodiments without departing from the spirit and scope of the claims and their equivalents. For example, suitable results may be achieved if the described techniques are performed in a different order, and/or if described components are combined in a different manner, and/or replaced or supplemented by other components or their equivalents.

Therefore, other implementations, other embodiments, and equivalents to the claims are also within the scope of the following claims.

## Claims

1. A nicotine pouch comprising:
a filler; and
a packaging material that wraps the filler,
wherein the filler comprises:
nicotine;
a release control excipient comprising at least one or more selected from a group consisting of cellulose and sugar alcohol;
a binder;
a pH adjuster; and
a flavoring agent,
wherein a content of the nicotine in the filler is 2.5 to 10 wt% with respect to a weight of total solid contents of the filler,
wherein a content of the release control excipient in the filler is 5.5 to 36 times the content of nicotine, and
wherein the packaging material comprises at least two or more selected from a group consisting of pulp, a cellulosic staple fiber, and a thermoplastic fiber.

2. The nicotine pouch of claim 1, wherein the cellulosic staple fiber comprises one or more selected from a group consisting of rayon, viscose rayon, and lyocell.

3. The nicotine pouch of claim 1, wherein the thermoplastic fiber comprises one or more selected from a group consisting of polypropylene (PP), polyethylene (PE), polyethylene terephthalate (PET), and polypropylene/polyethylene (PP/PE).

4. The nicotine pouch of claim 1, wherein the packaging material further comprises one or more binders selected from a group consisting of polyvinyl alcohol (PVA), an acrylic binder, polylactic acid (PLA), polybutylene succinate (PBS), polyhydroxyalkanoates (PHA), a vinyl acetate copolymer, a vinyl acrylic copolymer, a styrene-butadiene copolymer, and a plant-derived natural material.

5. The nicotine pouch of claim 1, wherein the packaging material comprises 40 to 70 wt% of the pulp and 30 to 60 wt% of the thermoplastic fiber.

6. The nicotine pouch of claim 1, wherein the packaging material comprises 40 to 70 wt% of viscose rayon, 30 to 60 wt% of the thermoplastic fiber, and 0 to 15 wt% of the binder.

7. The nicotine pouch of claim 1, wherein
the packaging material is prepared by a spun-lace method, and
the packaging material comprises 40 to 70 wt% of viscose rayon, 30 to 60 wt% of the thermoplastic fiber, and 0 to 15 wt% of the binder.

8. The nicotine pouch of claim 1, wherein
the packaging material is prepared by a wet laid method, and
the packaging material comprises 40 to 70 wt% of the pulp and 30 to 60 wt% of the thermoplastic fiber.

9. The nicotine pouch of claim 1, wherein the packaging material has a basis weight of 20 to 40 gsm, and a thickness of 50 to 250 µm.

10. The nicotine pouch of claim 1, wherein the packaging material has a porosity of 15,000 to 30,000, and a tensile strength of 1.00 to 1.50 N/mm.

11. The nicotine pouch of claim 1, wherein
a content of the cellulose in the filler is 1 to 2 times the content of the nicotine,
the content of the cellulose is 5 to 20 wt% with respect to the weight of total solid contents of the filler, and
a content of the sugar alcohol is 50 to 70 wt% with respect to the weight of total solid contents of the filler.

12. The nicotine pouch of claim 1, wherein
the pH adjuster of the filler comprises sodium bicarbonate and sodium carbonate, and
a weight ratio of the sodium bicarbonate to sodium carbonate is 7:3 to 6:4.

13. The nicotine pouch of claim 1, wherein a pH of the filler is 8.3 to 8.5.

14. A method of preparing the nicotine pouch of claim 1, the method comprising:
forming a web in a form of a sheet with a fiber raw material;
forming a nonwoven fabric by spraying moisture onto the web such that fibers are tangled;
preparing a packaging material by drying the nonwoven fabric; and
packaging a filler using the prepared packaging material.

15. A method of preparing the nicotine pouch of claim 1, the method comprising:
preparing a dispersion by dispersing a fiber raw material in water;
forming a web by moving the dispersion to a paper machine;
preparing a packaging material by drying the formed web; and
packaging a filler using the prepared packaging material.
